(19) Europäisches Patentamt
European Patent Office
Office européen des brevets

(11) Publication number: **0 038 153**
**A2**

(12) **EUROPEAN PATENT APPLICATION**

(21) Application number: **81301475.0**

(22) Date of filing: **06.04.81**

(51) Int. Cl.³: **A 61 K 39/35**

(30) Priority: **15.04.80 GB 8012295**

(43) Date of publication of application: **21.10.81**
**Bulletin 81/42**

(84) Designated Contracting States: **BE CH DE FR GB IT LI NL SE**

(71) Applicant: **BEECHAM GROUP LIMITED, Beecham House Great West Road, Brentford, Middlesex (GB)**

(72) Inventor: **Moran, David Martin, 1 Durnsford Way, Cranleigh Surrey (GB)**
Inventor: **Roy, Peter, Berkenlaan 47, NL-5941 EA Velden (NL)**

(74) Representative: **Dawson, Hugh Bainforde, Dr. et al, European Patent Attorney BEECHAM PHARMACEUTICALS Yew Tree Bottom Road, Epsom, Surrey KT18 5XQ. (GB)**

(54) **Modified allergens.**

(57) Allergens modified with oligomers consisting of 1 to 5 hydrophobic amino acids, pharmaceutical compositions containing them, a process for their preparation, and their use in the treatment of allergies.

**EP 0 038 153 A2**

# MODIFIED ALLERGENS

This invention relates to modified allergens, to a process for their preparation, and to their use in the therapy of allergic humans.

Many people are allergic to allergenic materials such as pollens, weeds and house dust. Such allergies have been conventionally treated by the administration to the sufferer of repeated gradually increasing doses of the relevant allergen, to build up resistance to the allergen. This is known as desensitisation.

In our UK Patent No 1 282 163 is described one improved form of therapy, in which the allergenicity of a given allergen is reduced by treatment with glutaraldehyde. It is found that such material maintains its ability to stimulate the desired blocking antibody, and thus may be used in desenitisation therapy with a reduced risk of side effects.

An alternative approach to the problem is the modification of the allergen such that on administration it suppresses the production of IgE antibodies specific to the unmodified allergen. Such an approach

is described in Dutch Patent Application No 7 709 025 (now also published as UK Patent No 1 578 348) wherein it is stated that allergen-polyethylene glycol conjugates are capable of eliciting the therapeutically desirable effect of suppressing in particular allergen specific IgE production. These materials are also stated to be substantially non-allergenic and non-immunogenic.

It has now been discovered that allergens may be modified, with a narrow class of peptides, to render them capable of suppressing allergen specific IgE antibody production.

Nowhere in the Dutch Patent Application referred to above is the use of this narrow class of peptides in any way disclosed or suggested.

Accordingly the present invention provides an allergen having bound thereto an oligomer consisting of 1 to 5 hydrophobic amino acids.

The allergen will be in the form of an extract of whole allergen, as is conventional. Suitable whole allergens from which the extract can be obtained include pollens, such as grass pollens for example rye; weeds, such as ragweed; house dust mites; venoms, such as bee-venom. Often the whole allergen will be ragweed, or a mixture of grasses.

The techniques for binding molecules to allergens are well known in the art, for example as described in the Dutch Patent Application. Basically, the binding depends on reacting active groups on allergen molecules with active groups on the oligomers, and if necessary or convenient providing such active groups as a first step.

By way of example, we have found that oligomers may conveniently be bound to allergens by bridges of formula (I):

[Allergen residue]-CO-X-CO- [Oligomer residue]

(I)

wherein X is a hydrocarbon chain of 1 to 4 carbons, optionally containing a double bond. The group -CO-X-CO- is suitably joined to an amino group on the allergen, and to an amino group on the oligomer.

Examples of suitable bridges include $-CO-CH_2-CO-$, $-CO-CH_2CH_2-CO-$ and -CO-CH=CH-CO-.

The oligomers for use in this invention must consist of 1 to 5 hydrophobic amino acids, and may be represented $A-(B)_m-(C)_n-(D)_o-(E)_p$ wherein each of A, B, C, D and E is a residue of a hydrophobic amino acid, and m, n, o and p are independently 0 or 1. Examples of suitable hydrophobic amino acids include those notionally derived from alanine β-substituted by an aromatic or aliphatic hydrophobic group, such as phenylalanine (Phe), valine (Val), leucine (Leu), iso-leucine (Ile) and norleucine (Nle). Conveniently the oligomer will consist of identical amino acids, preferably in the form of a dimer.

Suitable examples of the oligomer include peptides such as Ile-Ile, Phe-Phe and Val-Val.

Often the amino acids will be in their L-form.

It will be appreciated by the skilled worker that the oligomers may have their terminal carboxyl group modified, as is conventional in the peptide art. For example the terminal carboxyl group may be esterified or amidified for example in the form of a $C_{1-4}$ alkyl ester, or in the form of an amide, or mono- or di-$C_{1-4}$ alkyl amide.

From the aforesaid it will be appreciated that a particularly suitable material according to this invention is an allergen having a peptide A-B-Y, wherein A-B is Ile-Ile, Phe-Phe or Val-Val and Y is hydroxyl or methoxyl, bound thereto by bridges of formula -CO-X-CO-, wherein X is as defined.

In such materials suitable and preferred exmaples of the variables are as described.

Preferred materials according to the invention are those wherein the allergen is a pollen extract, the group X is $-CH_2-$ or $-CH_2CH_2-$ and the peptide is Ile-Ile-Y' wherein Y' is hydroxyl or methoxy.

It will be appreciated by the skilled worker that each allergen molecule will have a number of suitable sites for oligomer binding.

It will be a routine matter for the skilled worker, now we have discovered the advantages obtained thereby, to determine by simple experiment suitable binding levels for different allergens and different oligomers to give the desired (IgE suppression) activity. By way of illustration, we have found that suitably 10 to 70%, more suitably 10 to 40%, for example 20%, of the sites are bound. The level of site binding is easily determined for example as illustrated hereafter in the Examples.

Modified allergens according to this invention may, depending for example on the allergen, oligomer, and degree of binding, have a significant level of retained allergenicity (as well as the necessary IgE suppression activity). Such allergens form a useful class of materials within the invention. By way of example, which is not to be taken as limiting in any way, such materials may suitably have an allergenicity not less than 10% of the unmodified allergen.

In the art, materials consisting of allergens having bound thereto chemicals, such as for example the materials described in the said Dutch Patent Application, are often referrred to as "conjugates". Using this terminology, the materials of the present invention can be referred to as a conjugate of an allergen and of an oligomer consisting of 1 to 5 hydrophobic amino acids.

The materials of this invention suppress IgE production specific to the unmodified allergen. They may therefore be used in the therapy of allergy in humans. For example, if a patient is allergic to ragweed, then a material according to this invention in which the allergen is ragweed would be used.

The materials of the present invention may be employed as the active agents in compositions such as vaccines. Such compositions are well known to those skilled in the art and comprise a sterile liquid vehicle in which the active agent is dissolved or suspended, for administration by injection. If suspended, the particles of active agent should be small enough not to block the orifice of an injection needle. Certain additives such as tyrosine are often included in such compositions and are believed to provide a support and prolonged slow release of active material *in vivo*.

Usually a patient receiving treatment with such a composition is administered a number of injections, spread over a period of weeks or days.

It is also believed that the materials of the invention may be active via other routes of administration, such as to the nasal mucosa as a liquid spray or as a dry powder.

Accordingly in an additional aspect the invention provides a pharmaceutical composition comprising a material of the invention together with a pharmaceutically acceptable carrier.

A preferred composition of this invention is as a vaccine.

The compositions of this invention may, by way of illustration, suitably contain from 1 to 10,000 PNU of modified material. Normally of course doses towards the lower end of this scale will be more suitable for early in the therapy; doses towards the higher end of this scale for later in the therapy.

The invention also provides a process for the preparation of the materials of the invention, which process comprises binding the oligomer to the allergen.

This binding reaction can be carried out in any suitable manner, following long established procedures.

For example the oligomer may be reacted with a compound of formula M-X-Q wherein X is as defined, Q is a group reactable with amino group on the oligomer, and M is a group reactable with a group on the allergen.

Suitable examples of Q include the carboxyl group. Suitable examples of M include carboxyl, which group is suitably protected during binding of the Q group to the oligomer. Alternative examples of the compound M-X-Q include cyclic anhydrides such as for example

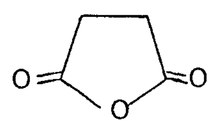

The thus formed M-X-Q- oligomer can be bound to the allergen for example by first activating the M group, for example when M is carboxyl suitably it is converted to its N-hydroxysuccinimide ester, and then reacting it with the allergen.

This reacting with the allergen of the activated oligomer is suitably achieved in common alkaline buffer systems for example phosphate, borate or bicarbonate, preferably in the pH range 7.5-9.0; typically a protein concentration in the range of 2.5 to 10 mg/ml is used. Reactions are suitably carried out between 4°C and ambient temperature for periods up to 24 hours.

The oligomers themselves are either available commercially or can be prepared following standard peptide synthetic techniques.

The degree of allergen substitution is generally controlled by adjusting the ratio of activated oligomer to allergen employed in the reaction. Unreacted activated oligomers and low molecular weight hydrolysis products may suitably be separated by dialysis or alternative de-salting procedures, such as gel filtration or ion exchange.

The following Examples illustrate the invention.

In these Examples the amino acids used were in the L-form.

Example A

Preparation of Rye-$COCH_2CO$-IleIleOMe

$CO_2H$

A Synthesis of active ester $CH_2$-CO-IleIleOMe

$CO_2H$

(i) Monobenzyl malonic acid, $CH_2$-CO Bzl

Dibenzyl malonate (16.0 g, 56.3 mM) in benzyl alcohol (40 ml) was stirred at room temperature and treated with ground KOH (3.2 g, 57.2 mM). The solution became warm and soon formed a gel which was left to stand overnight. The gel was triturated with ether and the solid collected dissolved in water, and the aqueous solution washed with ether. The reaction solution was admixed with ether and acidified to pH 2.5. Ether extracts were dried and evaporated to leave an oil which crystallises *in vacuo* overnight (5.5 g, 50.3%), mp 48-50$^{\circ}$C. The $^1$H-nmr spectrum was consistent with structure.

(ii) $Bzl0_2C$ $CH_2CO$-IleIleOMe

The dipepide IleIleOMe. HCl (2.2 g, 7.5 mM) in anhydrous MDC (25 ml) was cooled to 0$^{\circ}$C, treated with an equivalent of $Et_3N$ (1.05 ml), followed by DDCI (1.60 g, slightly >1 equiv) in MDC (2 ml) and monobenzyl malonic acid (1.45 g, 1 equiv) in MDC (5 ml). The mixture was stirred with cooling for 3 hours, R.T. for 1 hour, filtered and filtrate evaporated *in vacuo* (3.8 g). The residue was extracted with boiling EtAc (30 ml), cooled and filtered to remove further DC Urea. The filtrate was

evaporated *in vacuo* to give product (2.4 g, 74%) which solidifies on standing. T.L.C. (silica) Rf. 0.64 (8% EtOH in $CHCl_3$; $I_2$ stain), with minor impurities; mp 104-6°C. The n.m.r. spectrum was consistent with structure.

(iii) $HO_2C\ CH_2CO$-Ile.IleOMe

The above benzyl ester (1.2 g) in 85% aqueous AcOH (100 ml) was hydrogenated overnight in the presence of 10% Pd/C catalyst (1.2 g) using a continuous flow of hydrogen. The filtrate was evaporated *in vacuo*, water added to the residue and the mixture re-evaporated *in vacuo* and dried overnight (1.05 g). The product was purified by silica gel chromatography (Type 60H; 20 g) eluting with 10% MeOH in $CHCl_3$. Yield 0.65 g (68%) T.L.C. (20% MeOH in $CHCl_3/I_2$ stain) Rf. 0.45. The n.m.r. spectrum was consistent with structure.

(iv) 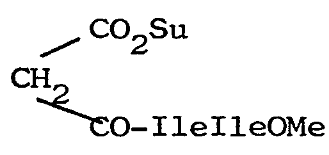

A solution of (iii) above (0.63 g, 1.83mM) and HOSu (0.21 g, 1 equiv.) in dioxan (30 ml) was cooled and treated with DCCI (0.41 g, 10% excess of equivalent). The reaction was stirred with cooling for 3 hours, R.T. for 2 hours, treated with AcOH (0.5 ml) and stirred for a further ½ hour. The cooled reaction mixture was filtered and the filtrate evaporated *in vacuo*. The residue was crystallised from IPA (15 ml) to give product (50 mg) mp 133-5°C. The crystallisation filtrate was diluted with petrol (80-100°) to give further

product (140 mg, total yield 24%). T.L.C. (5% MeOH in EtAc/$I_2$) showed product at Rf. 0.05 with minor mobile contaminant. The amino acid analysis trace showed Ile only. The n.m.r. spectrum was consistent with structure.

B Rye-$COCH_2CO$-IleIleOMe

Rye extract (25 mg) in borate buffer (pH 8.5, 0.1M, 2.5 ml) was cooled to ice temperature then treated with various amounts of the active ester, $SuO_2C.CH_2CO$-IleIleOMe (see Table 1) in acetone (0.25 ml). Precipitation occured at higher concentrations of active ester but clears up on reaction.

All samples were stirred for 15 minutes at $0^o$, 2 hours at R.T. dialysed exhaustively against 2mM $NH_4HCO_3$, and freeze dried.

By this method were prepared Samples 1 to 4 in Table 1.

Also prepared by this method were Samples 5 to 7 in Table 1.

Example B

Preparation of Rye-$CO(CH_2)_2$COIleIleOMe

(i) IleIleOMeHCl

IleOMeHCl (5 g, 0.0275 m) was dissolved in DMF (150 $cm^3$) with triethylamine (3.1 g). After cooling to $0^oC$, BocIleOSu (9.01 g) was added and the solution stirred overnight at room temperature. The reaction mixture was poured into water (300 $cm^3$) and extracted with ethyl acetate (3 x 50 $cm^3$), the combined extracts being subsequently washed with water and dried over $MgSO_4$. Solvent evaporation gave a pale viscous oil which was taken up in 2N HCl in ethyl acetate (200 $cm^3$) and stirred at room temperature for 2 hours. Evaporation of the solvent gave a yellow oil which was purified by precipitation from a chloroform solution with petroleum ether (40-60 bp). Yield 6.2 g, 76.5%.

(ii) $HOOC(CH_2)_2$COIleIleOMe

IleIleOMeHCl (6.2 g, 0.021 m) was dissolved in ethyl acetate (20 $cm^3$) and DMF (100 $cm^3$) together with succinic anhydride (2.1 g, 1 eq.) and triethylamine (3.0 g, 1.1 eq.). The solution was warmed on a steam bath for 10 minutes and stirred overnight at room temperature. The reaction mixture was then poured into water (400 $cm^3$) acidifed to pH 2 and extracted with ethyl acetate (3 x 50 $cm^3$), the combined extracts were washed with water and dried over $MgSO_4$. Solvent evaporation gave a white solid. Yield 6.33 g, 84%. N.m.r. was consistent with structure.

(iii) 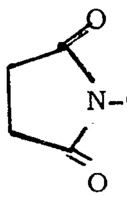 $-O-CO(CH_2)_2CO$ IleIleOMe

$HOCO(CH_2)_2COIleIleOMe$ (6.33 g, 0.0177 m) was dissolved in dioxan (150 $cm^3$) together with N hydroxy succinimide (HOSu) (2.0 g) and, after mild cooling, DCCI (3.65 g) in dioxan (20 $cm^3$) was added. The solution was stirred overnight at room temperature, acidified with glacial acetic acid (1 $cm^3$), subsequently being cooled and filtered. Evaporation of the solvent and recrystallisation gave a white solid. Yield 7g, 87%. N.m.r. consistent with structure.

(iv) <u>Rye-$CO(CH_2)_2COIleIleOMe$</u>

Rye grass extract (30 mg) was dissolved in borate buffer (0.1 M, 3 ml, pH 8.5) at $0^oC$. Amounts of active ester dissolved in acetone (0.6 $cm^3$) and water (0.1 $cm^3$) as shown in Table 1 were then added and the solutions stirred at $0^oC$ for 15 minutes.

After a further 2 hours stirring at room temperature the samples were dialysed exhaustively against $NH_4HCO_3$ (10 mmol) then freeze dried.

By this method was prepared Samples 12 and 13 in Table 1.

| List of Abbreviations used in Examples A & B | |
|---|---|
| MDC | Dichloromethane |
| DCCI | Dicyclohexyl carbodi-imide |
| EtAc | Ethyl acetate |
| DC Urea | Dicyclohexyl urea |
| IPA | Iso-propyl alcohol |

## Example 1

### Preparation of methyl N-(2-succinimidyloxycarbonylacetyl)-isoleucinylisoleucinate, $SuO_2CCH_2CO$-IleIleOMe

#### (i) Synthesis of benzyloxycarbonylacetic acid, $BzlO_2CCH_2CO_2H$

To a stirred mixture of dibenzyl malonate (16.0 g, 56.3 mmole) and benzyl alcohol (40 ml) was added finely ground potassium hydroxide (3.2 g, 57.2 mmole). The mixture became warm and formed a gel which was left overnight. It was then triturated with diethyl ether and the residual solid collected by filtration and dissolved in water. After washing with more diethyl ether the aqueous solution was acidified to pH 2.5 and extracted with diethyl ether. These extracts were combined, dried over $MgSO_4$ and then concentrated to an oil after removal of the drying agent by filtration. This oil crystallised when left *in vacuo* overnight. Yield 5.5 g, 50.3%. Mpt 48-50°C.

$^1$H-nmr, $CDCl_3$ (TMS), δ, 3.50 (s, 2H, $CH_2$), 5.18 (s, 2H, Ph$CH_2$), 7.34 (s, 5H, Ph), 11.34 (s, 1H, $CO_2H$).

#### (ii) Synthesis of methyl isoleucinylisoleucinate hydrochloride, HIleIleOMe.HCl

Methyl isoleucinate hydrochloride (5 g, 27.5 mmole) was dissolved in N,N-dimethylformamide (DMF)(150 ml) and treated with triethylamine (3.82 ml, 27.5 mmole).

The suspension was cooled in an ice-water bath and succinimidyl N-t-butyloxycarbonylisoleucinate, BOCIleOSu (9.01 g, 27.5 mmole) added. The mixture was stirred at ~4°C for 30 minutes then at room temperature overnight. It was then poured into iced-water (300 ml) which was extracted several times with ethylacetate (3 x 50 ml). The combined extracts were washed with saturated brine, dried over $MgSO_4$ and concentrated to a pale viscous oil, assumed to be BOCIleIleOMe. This was taken up in pre-cooled (~4°C) 3-N-HCl in ethyl acetate (200 ml) and stirred for 2 hours at room temperature. Evaporation of the solution gave a slightly yellow oil which was taken up in chloroform and a white solid was precipitated by the addition of petroleum ether (bp 40-60°). Yield 6.2 g, 76.5%.

$^1$H-nmr, $CDCl_3$ (TMS), δ, 0.7-2.3 (m, 18H, 2 (γ $CH_2$, 2 $CH_3$, β CH)), 3.80 (s, 3H, $OCH_3$), 4.1-4.6 (m, 2H, 2 (αCH)), 7.9 (m, 1H, NH), 8.0-8.7 (bs, 3H, $NH_3^+$).

(iii) Synthesis of methyl N-(2-benzyloxycarbonyl)-isoleucinylisoleucinate, $BzlO_2CCH_2COIleIleOMe$

The dipeptide hydrochloride prepared above (2.2 g, 7.5 mmole) suspended in dry methylene chloride (25 ml) was cooled in an ice-water bath and treated with an equivalent of triethylamine (1.05 ml, 7.5 mmole) followed by dicyclohexylcarbodiimide (1.6 g, 7.75 mmole) in methylene chloride (2 ml) and benzyloxycarbonylacetic acid (1.45 g, 7.6 mmole) in methylene chloride (5 ml). The mixture was stirred at ~4°C for 3 hours then at room temperature for 1 hour. It was then filtered and the

filtrate concentrated *in vacuo*. The residue was extracted with a small volume of warm ethyl acetate (30 ml), the solution cooled and filtered. This was dried over $MgSO_4$ and concentrated to a solid, yield 2.4 g, 76%. Mpt 108-10$^{o}$; TLC (silica, EtOH/$CHCl_3$, (8/92), $I_2$ stain) $R_f$ 0.64.

$^1$H-nmr, $CDCl_3$ (TMS), δ, 0.7-2.3 (m, 18H, 2 (γ $CH_2$, 2($CH_3$), β CH)), 3.40 (s, 2H, -$CH_2$), 3.80 (s, 3H, $OCH_3$), 4.2-4.8 (m, 2H, 2 (α CH)), 5.20 (s, 2H, Ph$CH_2$), 6.3-6.6 (m, 1H, NH), 7.2-7.4 (m, 1H, NH), 7.30 (s, 5H, Ph).

(iv) Synthesis of methyl *N*-(2-carboxyacetyl)-isoleucinyliso leucinate, $HO_2CCH_2COIleIleOMe$

The benzyl ester prepared above (3.0 g, 6.9 mmole) was dissolved in 85% aqueous acetic acid (200 ml) and hydrogenated at room temperature and pressure for 5 hours over 10% Pd/C (3 g). TLC (silica, MeOH/$CHCl_3$, (2/8)) indicated the reaction to be complete at this time so the reaction mixture was filtered through Celite and the filtrate concentrated at reduced pressure. The residue was taken up in water and the solution evaporated to give a white solid after drying over $P_2O_5$ *in vacuo* overnight, yield 1.8 g, 76%. TLC indicated this to be homogenous, $R_f$ 0.45, so it was used without further purification.

(v) Synthesis of methyl N-(2-succinimidyloxycarbonyl-acetyl)-isoleucinyl-isoleucinate, $SuO_2CCH_2CO$ Ile IleOMe.

A solution of the half ester prepared in (iv) (0.63 g, 1.83 mmole) and N-hydroxysuccinimide (0.21 g, 1.83 mmole) in dioxan (30 ml) was carefully cooled and treated with dicyclohexylcarbodiimide (0.41 g, 1.89 mmole). The mixture was stirred for 3 hours while being cooled and 2 hours at room temperature. Acetic acid (0.5 ml) was added and stirring continued for 30 minutes. The reaction mixture was filtered and the filtrate evaporated at reduced pressure. The residue was crystallised from chloroform/diethyl ether, yield 0.21 g, 27%. Mpt 159-61°.

Microanalysis: $C_{20}H_{31}N_3O_8$ requires: C, 54.41; H, 7.08; N, 9.52: Found C, 54.39; H, 7.34; N, 9.47.

$^1$H-nmr, $CDCl_3$ (TMS), δ, 0.8-2.3 (m, 18H, 2(γ $CH_2$, 2 ($CH_3$), β CH); 2.90 (s, 4H, OSu); 3.70 (s, 2H, $CH_2$); 3.80 (s, 3H, $OCH_3$); 4.2-4.8 (m, 2H, 2 (αCH)); 6.3-6.7 (m, 1H, NH); 7.1-7.5 (m, 1H, NH).

Example 2

Preparation of Rye-$COCH_2CO$IleIleOMe conjugates

Rye grass pollen extract (30 mg) was dissolved in borate buffer (3 ml, 0.1 M, pH 8.5) and the solution cooled in an ice-water bath. To this solution was added an acetone/water solution (0.2/0.1ml) of the appropriate quantity (see Table 1) of the activated dipeptide derivative prepared in Example 1(v). After stirring for 15 minutes the cooling bath was removed and stirring continued for 2 hours at room temperature. The solutions were then dialysed exhaustively against 10 mmole $NH_4HCO_3$ at $5^o$ and subsequently lyophilised.

The products were characterised by amino acid analysis and/or, primary amino group content (by a standard assay using 2,4,6-tri-nitrobenzenesulphonic acid).

Sample Nos 8-11 (Table 1).

Example 3

Preparation of Ragweed-$COCH_2COIleIleOMe$

These were prepared in a similar manner to that described in Example 2 except that ragweed pollen extract (30mg) was used in place of rye grass pollen extract.

Samples 34 - 38 (Table 2)

## Example 4

**Preparation of methyl N-(3-succinimidyloxycarbonylpropionyl)-isoleucinyl-isoleucinate, $SuOOCCH_2CH_2COIleIleOMe$**

The dipeptide hydrochloride prepared in Example 1, (ii) (6.2g, 21 mmole) was dissolved in DMF (100ml) and treated with triethylamine (4.12ml, 29.6 mmole) followed by succinic anhydride (2.1g, 21 mmole). The solution was warmed on a steam bath for 10mins then allowed to stand at room temperature overnight. The mixture was poured into water (400ml) which was extracted with ethyl acetate (3 x 50ml) after acidification to pH 2.0. The combined extracts were washed with water, dried over $MgSO_4$ and concentrated to a white solid. Yield 6.33g, 84%. The $^1H$ nmr spectrum of this material was consistant with its being the desired compound, methyl N(3-carboxypropionyl)-isoleucinylisoleucinate, so it was used without further purification. It was dissolved in dioxan (150ml) together with N-hydroxysuccinimide (2.0g, 1.98 mmole) and the solution carefully cooled in an ice-water bath before dicyclohexylcarbodiimide(3.65g, 1.78 mmole) in dioxan (20ml) was added. After stirring for 15 mins in the ice-bath the mixture was allowed to warm to room temperature and stirred overnight. It was then filtered and the filtrate concentrated at reduced pressure. Recrystallisation from chloroform/pet. ether (bpt. 40-60$^o$) gave a white solid, 7g, 87%. $^1H$-nmr, $CDCl_3$(TMS), $\delta$, 0.6-2.0(M,18H,2($\gamma CH_2$,2($CH_3$),$\beta$CH),2.80 (S,4H,OSu),2.80(m,4H,$CH_2CH_2$), 3.70(s,3H,$OCH_3$), 4.40 (m,2H,2($\alpha$CH)), 6.50(m,2H,2NH)

Example 5

Preparation of Rye-$COCH_2CH_2CO$IleIleOMe conjugates

These conjugates were prepared using the method described in Example 2 to react rye grass pollen extract with the activated dipeptide derivative described in Example 4. The proportions of each reactant used and the analytical data on the products are given in Table 1, Sample Nos. 14 and 15.

Example 6

Preparation of Ragweed-$COCH_2CH_2CO$IleIleOMe conjugates

These conjugates were prepared by the method described in Example 3 using the activated dipeptide derivative prepared in Example 4 in the proportions shown in Table 2, Sample Nos. 31 -33 .

Example 7

Preparations of Rye-COCH=CHCOIleIleOMe conjugates.

(i) Synthesis of methyl N-(3-succinimidyloxycarbonylprop-2,3-enoyl)-isoleucinyl-isoleucinate, $SuO_2CCH$=CHCOIleIleOMe

The dipeptide hydrochloride prepared in Example 1,(ii) was converted to $SuO_2CCH$=CHCOIleIleOMe via methyl N-(3-carboxyprop-2,3-enoyl)-isoleucinylisoleucinate, $HO_2C$-CH=CHCOIleIleOMe, using procedures similar to those described in Example 4 employing maleic anhydride in place of succinic anhydride.

(ii) Rye-COCH=CHCOIleIleOMe

This was prepared from the activated dipeptide derivative prepared above using a procedure similar to that described in Example 2. Sample Nos. 16 and 17, Table 1.

Example 8

Preparation of Rye-$COCH_2CH_2COIleOMe$ conjugates

The requisite activated derivative methyl N-(3-succinimidyloxycarbonyl propionyl)-isoleucinate, $SuO_2CCH_2CH_2COIleOMe$ was prepared by using the procedures described in Example 4 on methyl isoleucinate hydrochloride in place of methyl isoleucinylisoleucinate. The product was then coupled to rye grass pollen extract by the method described in Example 2. Sample Nos. 18 and 19 in Table 1.

Example 9

Preparation of Rye-$COCH_2CH_2$COIleIleIleOMe conjugates

Synthesis of methyl N-(3-succinimidyloxycarbonyl propionyl) isoleucinyl-isoleucinylisoleucinate, $SuO_2CH_2CH_2$COIleIleIleOMe

The N-t-butyloxycarbonyl protected dipeptide , BOCIleIleOMe, prepared as an intermediate in Example 1, (ii), (2.58g, 7.2 mmole) was dissolved in dioxan (100 ml) and stirred with aqueous sodium hydroxide (10 ml, 3M, 30 mmole) for several hours at room temperature. The reaction mixture was then partitioned between water and chloroform (500 ml each) and the aqueous phase was brought to pH 2.0. The aqueous was extracted with more chloroform (2 x 50 ml) and the combined extracts washed with water several times, dried over $MgSO_4$ and concentrated to a white solid, yield 2.18g, 88% $^1$H nmr, $CDCl_3$(TMS), $\delta$, 0.6-2.3(m,18H 2($\gamma CH_2$,2($CH_3$),$\beta$CH)),1.45(S,9H,$(CH_3)_3C$),4.03(t,1H,$\alpha$H), 4.63(t,1H,$\alpha$H),5.50(br.d.,1H,NH),6.95(br.d.,1H,NH), 8.00 (br.s.,1H,$CO_2$H), was sufficient to identify this as N-(t-butyloxcarbonyl)isoleucinyl-isoleucine, BOCIleIleOH, which was used without further purification. Thus, BOCIleIleOH (1.73g, 5 mmole) was dissolved in chloroform (40 ml) together with methyl isoleucinate hydrochloride (1g, 5.5 mmole) and triethylamine (0.4 ml, 5.5 mmole) the mixture cooled in an ice-water bath and treated with dicyclohexylcarbodi - imide (1.14g, 5.5mmole). The reaction was stirred at room temperature overnight, then filtered and the filtrate washed sequentially with 5% aqueous citric acid and sodium bicarbonate. After drying over $MgSO_4$ the organic phase was evaporated to a white solid, yield 0.5g, 21%. This was identified as BOCIleIleIleOMe

from its $^1$Hnmr spectrum, $CDCl_3$(TMS),$\delta$,0.6-2.3(m,27H, 3($\gamma CH_2$,2($CH_3$),βCH)),1.45(S,9H,$(CH_3)_3C$),3,70(S,3H,$OCH_3$), 3.65-4.70(m,3H,3(αCH)),5.2(br.d.,1H,NH),6.67(br.m.,1H,NH). This was dissolved in 3N-HCl in ethyl acetate and stirred for 3 hours at room temperature. Excess diethyl ether was added and the precipitated solid collected by filtration. This was dissolved in chloroform (20 ml) and succinic anhydride (0.21g,2.12 mmole) and triethylamine (0.3 ml, 2.12 mmole) added. The mixture was heated on the steam bath for 3 mins then stirred at room temperature for 2 hours. The solution was washed with dilute aqueous acetic acid containing about 5% methanol by volume. The lower, organic phase was dried over $MgSO_4$ and concentrated to a white solid, yield 0.45 g, 89%. $^1$Hnmr,$CD_3OD$(TMS),$\delta$,0.3-2.15 (br.m.,27H,3($\gamma CH_2$,2($CH_3$),βCH)),2.53(br.s.,4H,$CH_2CH_2$), 3.70( s,3H,$OCH_3$),4.25(br.m.,3H,3(αCH)) identified this as methyl N-(3-succinimidyloxycarbonylpropionyl)-isoleucinylisoleucinylisoleucinate, $HO_2CCH_2CH_2$COIleIleIleOMe which was converted to its succinimido ester as follows. The solid (0.313g, 0.66 mmole) was dissolved in DMF (12 ml) with N-hydroxysuccinimide (0.115g, 0.99 mmole) and the solution cooled in ice-water before dicyclohexylcarbodiimide (0.177 g, 0.86 mmole) was added. The mixture was allowed to warm to room temperature during 3 hours stirring whereupon 2 drops of acetic acid were added. After 30 mins the suspension was filtered and the filtrate concentrated under high vacuum (<30°). The residue was dissolved in chloroform the solution filtered and washed with water several times before drying over $MgSO_4$ and concentration. The residue was crystallised from chloroform/diethyl ether, yield 0.296 g, 79%. Mpt. 185-7°. $^1$Hnmr,DMSO(TMS),$\delta$,0.3-2.1(m,27H,3($\gamma CH_2$, 2($CH_3$),βCH)),2.2-3.0(br.m.,4H,$CH_2CH_2$) ,2.76(S,4H,OSu),3.56 (S,3H,$OCH_3$),4.20(br.m.,3H,3(αCH)),8.0(br.m.,3H,3(NH)).

(ii) Rye-$COCH_2CH_2$COIleIleIleOMe

Conjugates were then prepared from the tripeptide methyl ester described above by procedures similar to those described in Example 2. Proportions of activated tripeptide used are given below in Table 1, Sample Nos. 20 - 22.

Example 10

The following dipeptide methyl ester hydrochlorides were prepared by standard peptide synthesis methodology, as illustrated by the description in Example 1, (ii), from the appropriate N-t-butyloxycarbonylamino acid succinimido ester and amino acid methyl ester :-

HPhePheOMe.HCl
HValValOMe,HCl
HGlyGlyOMe,HCl

Each of these compounds was then reacted with succinic anhydride and the product converted to the succinimido active ester using the methods described in Example 4. The resulting activated dipeptide esters were coupled to rye grass pollen extract under the conditions described in Example 5. Sample Nos. 23 - 30, Table 1.

TABLE 1

Preparative and analytical data on rye grass pollen extract modified with hydrophobic peptides

| Example No | Sample No | Wt ratio peptide/rye extract | % $1^{o}NH_2$ modification |
|---|---|---|---|
| A | 1 | 2/1 | 56.6 |
| | 2 | 1/1 | 47.1 |
| | 3 | 0.5/1 | 20.4 |
| | 4 | 0/1 | 0 |
| | 5 | 1/1 | 44.0 |
| | 6 | 0.5/1 | 18.0 |
| | 7 | 0/1 | 0 |
| 2 | 8 | 0/1 | 0 |
| | 9 | 0.034/1 | 1 |
| | 10 | 0.17/1 | 12 |
| | 11 | 0.48/1 | 22 |
| B | 12 | 0/1 | 0 |
| | 13 | 0.1/1 | 14.8 |

- 31 -

Table 1 cont ...

| Example No | Sample No | Wt ratio peptide/rye extract | % $1^{O}NH_2$ modification |
|---|---|---|---|
| 5 | 14 | 0/1 | 0 |
| | 15 | 0.33/1 | 21 |
| 7 | 16 | 0/1 | 0 |
| | 17 | 0.2/1 | 26.9 |
| 8 | 18 | 0/1 | 0 |
| | 19 | 0.2/1 | 41.5 |
| 9 | 20 | 0/1 | 0 |
| | 21 | 0.033/1 | 6 |
| | 22 | 0.17/1 | 32 |

Table 1 cont ...

| Example No | Sample No | Wt ratio peptide/rye extract | % $1^{o}NH_2$ modification |
|---|---|---|---|
| 10 | 23 | 0 | 0 |
| | 24 | 0.2/1 | 29.3 |
| | 25 | 0 | 0 |
| | 26 | 0.2/1 | 36.1 |
| | 27 | 0 | 0 |
| | 28 | 0.2/1 | 3.0 |
| | 29 | 0.8/1 | 13.2 |
| | 30 | 2.0/1 | 34.0 |

TABLE 2

Preparative and analytical data on ragweed pollen extract modified with hydrophobic peptides.

| Example No | Sample No | Wt ratio peptide/ragweed extract | % $1^{O}NH_2$ modification |
|---|---|---|---|
| 3 | 34 | 0/1 | 0 |
| | 35 | 0.03/1 | 11 |
| | 36 | 0.18/1 | |
| | 37 | 0.54/1 | 23 |
| | 38 | 1.07/1 | 50 |
| 6 | 31 | 0/1 | 0 |
| | 32 | 0.19/1 | 17 |
| | 33 | 0.53/1 | 47 |

Suppression of the Developing IgE Response in BDI Mice

Method

Groups of 6-8 mice are all immunised intraperitoneally with 10 μg of antigen adsorbed onto 0.25 mg-1.0 mg of aluminium hydroxide gel.

On days 3, 5 and 7 the animals are injected intravenously with various amounts, usually 100 μg of the modified allergen under test, in 0.5 ml diluent, unmodified allergen or diluent (0.5 ml).

Serum is taken usually on days 10, 17 and 24. Serum from animals is bulked in groups at each bleeding time.

The antigen specific IgE antibody in each serum is titrated by a standard passive cutaneous anaphylaxis test in two rats using a latent period of 48 hours.

Results are expressed at $-log_4$ or $-log_2$ from ¼ of the last dilution giving a positive result.*

To show suppressive activity, materials when used for treatment, must reduce IgE levels in immunised animals to lower levels than in those animals immunised and treated with diluent or unmodified antigen.

Illustrative results obtained with materials prepared according to the invention are shown in Tables 3 and 4.

[Diluent (PBS) is Bacto-haemagglutination buffer (Difco)].

* [A result of 0 indicates a level of specific IgE below the limit of detection.]

TABLE 3

Suppression of the IgE response with rye-hydrophobic peptide modified allergens.

| Experiment No | Treatment i.v. | | Rye-Specific IgE $-\log_4$ from ¼ | | | | | |
|---|---|---|---|---|---|---|---|---|
| | Material Sample No/Other | Amount/μg | Day 10 | 17 | 24 | 31 | 38 | 45 |
| 1 | PBS | | >6 | 6 | 5 | 4.5 | 4.5 | 5 |
| | rye pollen extract | 100 | 4 | 1.5 | 1.5 | 3 | 2 | 2 |
| | buffer control rye (4) | " | 4 | 0.5 | 2 | 2.5 | 3 | 2.5 |
| | 1 | " | 3 | 1.5 | 2.5 | 2 | 1 | 0.5 |
| | 2 | " | 0 | 0 | 1.5 | 2 | 0 | 0 |
| | 3 | " | 2.5 | 0 | 0 | 0 | 0 | 0 |
| 2 | | | Day 10 | 21 | 26 | 34 | 55 | |
| | PBS | | 4 | 4 | 4 | 5 | 4 | |
| | buffer control rye (7) | 100 | 3.5 | 2 | 3 | 3 | 3.5 | |
| | 5 | " | 0 | 3 | 3 | 3 | 3 | |
| | 6 | " | 0.5 | 0 | 1 | 0 | 2 | |

Table 3 cont ...

| Experiment No | Treatment i.v. | | Rye-Specific IgE $-\log_2$ from ¼ | | | |
|---|---|---|---|---|---|---|
| | Material Sample No/Other | Amount µg | Day 17 | 24 | 38 | 54 |
| 3 | PBS | | 5.5 | 7 | 7 | 6 |
| | rye pollen extract | 100 | 0 | 1 | 0 | 2.5 |
| | buffer control rye (14) | " | 0.5 | 2.5 | 4 | 4.5 |
| | 15 | " | 0 | 0.5 | 1 | 0 |
| | | | Day 17 | 24 | 31 | |
| 4 | PBS | | 3 | 5 | | |
| | rye pollen extract | 100 | 0 | 1.5 | | |
| | buffer control rye (20) | " | 0 | 1 | | |
| | 21 | " | 0 | 0 | | |
| | 22 | " | 0 | 0.5 | | |

TABLE 4

Suppression of the IgE response with ragweed-hydrophobic peptide modified allergen.

| Experiment No | Treatment i.v. | | Rye-Specific IgE $-\log_2$ from ¼ | | | |
|---|---|---|---|---|---|---|
| | Material Sample No/Other | Amount /µg | Day 10 | 17 | 24 | 31 |
| 5 | PBS | | 0 | 0 | 3 | 4 |
| | ragweed pollen extract | 100 | 5 | 2.5 | 2 | 2 |
| | buffer control ragweed (31) | " | 5 | 2 | 2.5 | 2 |
| | 32 | " | 4 | 0 | 2 | 1 |
| | 33 | " | 0 | 0 | 0 | 0.5 |

Discussion of Results

These results show that the modified materials of the invention have the desired activity.

Toxicity

No toxic effects were observed in these tests.

What we claim is:

1. An allergen having bound thereto an oligomer consisting of 1 to 5 hydrophobic amino acids.

2. A material according to claim 1, wherein the allergen is an extract of ragweed, or of a mixture of grasses.

3. A material according to claim 1 or claim 2, wherein the oligomer consists of amino acids selected from phenylalanine, valine, leucine, *iso*-leucine and norleucine.

4. A material according to claim 1, 2 or 3, wherein the oligomer consists of two or three amino acids.

5. A material according to any one of the claims 1 to 4, wherein the oligomer is IleIle or IleIleIle in which the terminal carboxy group may be in the form of its $C_{1-4}$ alkyl ester.

6. A material according to any one of the claims 1 to 5, wherein the oligomer is bound to the allergen by a bridge of formula (I):

[Allergen residue] - CO - X - CO - [Oligomer residue]

wherein X is a hydrocarbon chain of 1 to 4 carbons optionally containing a double bond.

7. A material according to any one of the claims 1 to 6, wherein 10 to 40% of the sites on the allergen have bound thereto the said oligomer.

8. A material according to any one of the claims 1 to 7, having a retained allergenicity.

9. A material according to claim 1, wherein the allergen is ragweed or a mixture of grasses, the oligomer is IleIleOMe, the oligomer is bound to the allergen by $-OC-CH_2CH_2-CO-$ bridges, and about 20% of the sites on the allergen are so bound.

10. A pharmaceutical composition comprising a material according to any one of the claims 1 to 9 together with a pharmaceutically acceptable carrier.

11. A process for the preparation of a material according to claim 1, which process comprises binding the oligomer to the allergen.

12. A material according to any one of the claims 1 to 9 for use in the treatment of allergy.